Europäisches Patentamt

(19) Eurcpean Patent Office

Office européen des brevets

(11) Publication number: **0 049 606**

**A1**

(12) # EUROPEAN PATENT APPLICATION

(21) Application number: 81304542.4

(22) Date of filing: 01.10.81

(51) Int. Cl.³: **C 12 Q 1/00**
**C 12 Q 1/48**
**//G01N33/54**

(30) Priority: 02.10.80 EP 80303478
02.10.80 PC T/GB80/00153
31.03.81 GB 8110031
01.04.81 GB 8110233

(43) Date of publication of application:
14.04.82 Bulletin 82/15

(84) Designated Contracting States:
AT BE CH DE FR GB IT LI NL SE

(71) Applicant: Self, Colin Henry
46 Lensfield Road
Cambridge, CB2 1EG(GB)

(72) Inventor: Self, Colin Henry
46 Lensfield Road
Cambridge, CB2 1EG(GB)

(74) Representative: Cole, William Gwyn
The Shires 3 Cricketfield Lane
Bishops Stortford Hertfordshire(GB)

(54) Detection method, use and diagnostic aid.

(57) A method is described of detecting a catalyst or its substrate in a catalysable reaction which comprises carrying out an assay for the catalyst or its substrate in a manner which produces a product characterised in that said product is an activator for an enzymatic reaction which is activated by the production thereof so as to effect a detectable change and to produce a facilitator for the enzymatic reaction; whereby said enzymatic reaction is enhanced.

-1-

Detection Method, Use and Diagnostic Aid.

This invention relates to a method of detecting a catalyst or a substrate in a catalysable reaction and to the use of this method in the detection of clinically or environmentally important materials present in trace amounts.

The desirability of having a method which can detect trace amounts of a catalyst or its substrate which is convenient and does not need the use of radioactive substances or complex equipment is obvious. Known methods of detection, for example heterogenous specific binding assays such as that of British Patent No. 1548741 or homogenous specific binding assays such as that of British Patent No. 1552607 are useful but can suffer from a lack of sensitivity. A particularly sensitive assay has now been discovered which does not require the use of radioactive substances or complex equipment.

Accordingly the present invention provides a method of detecting a catalyst or its substrate in a catalysable reaction which comprises carrying out an assay for the catalyst or the substrate in a manner which produces a product which method is characterized in that said product is an activator for an enzymatic reaction which is activated by the production thereof so as to effect a detectable change and to produce a facilitator for the enzymatic reaction; whereby said enzymatic reaction is enhanced.

When used herein the term "activator" means a substance which is required for an enzymatic reaction to take place if all other components needed for that enzymatic reaction are present.

When used herein the term "facilitator" means a substance which is not an enzyme but which acts so as to increase the rate at which the detectable change takes place. The facilitator is most suitably a cofactor for the enzymatic reaction.

Normally and preferably the facilitator produced by the enzymatic reaction is the same as the activator produced by the assay for the catalyst or its substrate. When the facilitator and activator are the same a desirable simplification of the system occurs. The feedback of the facilitator into the earlier part of the reaction sequence causes a marked increase in the rate of formation of the detectable change.

When used herein the term "detectable change" means a change in the concentration of a moiety which can be observed. The observation can be by any method but most aptly it is by a change is the optical characteristics. The change in the optical characteristics may be observed by eye or with the aid of a device such as a spectrophotometer. The change in concentration observed may be the increase in concentration of a moiety produced by the enzymatic reaction or it may be the decrease in concentration of a moiety consumed by the enzymatic reaction.

The enzymatic reaction may use a single enzyme or more than one enzyme. If more than one enzyme is employed it will aptly be not more than four enzymes, it will more aptly be not more than three

0049606

-4-

and it will preferably be not more than two enzymes.

The catalyst to be detected may be an enzyme
or a non-enzymic catalyst.

Normally and preferably the catalyst to be
detected is an enzyme. Enzymes are often required
to be detected in clinical chemical or vetinary
investigations. Abnormal levels of enzymes can
be present in pathological conditions so that their
detection (either qualitatively or quantitatively)
can be used as a diagnostic aid or to monitor the
progress of the pathological state. Thus, for example,
it is desirable to detect such enzymes as kinases,
for example creatine .kinase (a marker for
breast cancer and of use in diagnosing myocardial
infarction and brain damage and the like) and pyruvate
kinase (for diagnosis and monitoring of liver & muscle
pathology) and dehydrogenases, for example
lactodehydrogenase (for diagnosing blood diseases
and liver diseases); and the like.

Alternatively the catalyst may be a non-enzymatic
catalyst such as an electron transfer reagent. The electron transfer reagent can be a molecule such as phenazine ethosulphate or its analogues or a quinone or the like and may be conjugated to another molecule if desired.

If the method of this invention is used to detect a substrate for a catalyst it is normally and preferably a substrate for an enzyme. Such materials can have clinical significance, for example pyridine nucleotides such as ATP, GTP, CTP, UTP and the like or creatine phosphate or the like. Such materials are often of interest in metabolic studies and studies of kidney function.

Naturally the terms activator and facilitator as used herein cannot be interpreted as relating to classical cycles such as those of the recognised prior art of BP 1548741 and 1552607.

The preferred enzymatic reaction for use in the amplification method of this invention comprise kinases or dehydrogenases.

A particularly apt enzymatic reaction employing kinases may be represented as follows:

In this system the production of ATP (produced by the assay and hence the "activator") enables the PKF to convert F6P to FDP. The thus produced FDP activates the PK(I) which converts the ADP present to ATP and the PEP to pyruvate (which causes a detectable change as explained hereinafter). The ATP produced by the PK(I) (the "facilitator") feeds back to allow the PFK to produce more FDP which increases the activity of the PK(I) which leads to the production of more ATP and pyruvate. Hence a marked increase in production of pyruvate occurs which in turn leads to a marked increase in the rate of detectable change.

An apt method of using the above enzymatic reaction to lead to a detectable change is to employ the following system:

$$\text{pyruvate} \searrow \quad \text{LDH} \quad \nearrow \text{NADH}$$
$$\text{lactate} \nearrow \qquad\qquad \searrow \text{NAD}$$

In this system the introduction of pyruvate causes the LDH to oxidize the NADH to NAD. This can then be monitored spectrophotometrically, for example at 340nm.

It will be appreciated that the preceeding enzymatic reaction can be adapted to the detection of a nucleotide triphosphate such as ATP or an enzyme that produces such a triphosphate or even a substrate for such an enzyme. Most desirably the preceeding enzymatic reaction is adapted to the detection of an enzyme which produces ATP. The preferred ATP producing enzymes are kinases which do not require FDP for their activation. Such enzymes include pyruvate kinase (PKII) and creatine kinase (CK) (sometimes also called creatine phosphokinase).

0049606

-8-

In the case of (PK II) a reaction leading to the production of the activator (ATP) is as follows:

$$\text{PEP} \diagdown \text{PK} \diagup \text{ADP}$$
$$\text{pyruvate} \diagup \qquad \diagdown \text{ATP}$$

In the case of CK a reaction leading to the production of the activator (ATP) is as follows:

$$\text{CP} \diagdown \text{CK} \diagup \text{ADP}$$
$$\text{C} \diagup \qquad \diagdown \text{ATP}$$

A particularly apt enzymatic reaction employing dehydrogenase may be represented as follows:

pyruvate — LDH — NADH
lactate — NAD

oxidized lipoamide — Diaphorase (NAD-free) — NADH
reduced lipoamide — NAD

Diaphorase requires NAD to function so that NAD-free diaphorase is activated by the NAD produced by the catalyst

The detectable change produced by this reaction is the production of NAD from NADH which may be monitored at 340nm.

The skilled worker will appreciate that the reactions hereinbefore discussed are biochemical reactions of the type that will be performed under substantially physiological conditions; that is in aqueous solution under non-extreme conditions, for example at a temperature of 5-40°C and more usually 15-37°C; at pH values of 5-9, more usually of 5.5-8.5 and preferably at approximately 7 (pH will generally be maintained in the desired range using a buffer such as DMG or imidazol buffer).

It is envisaged that normally all the reagents required for operation of the assay and enzymatic reactions will be present except the activator, facilitator and the material to be detected. The method of this operation comes into effect when the material to be detected is added.

In this specification the term "detecting" extends to quantitative detection as well as to non-quantitative detections; such "detecting" is often referred to as "determining".

In a broad aspect this invention provides a method of detecting an activator (as hereinbefore defined) which comprises contacting said activator with an enzyme to be activated and all other components needed for the enzymic reaction to proceed characterized in that said enzymatic reaction causes a detectable change and the production of a facilitator (as hereinbefore defined) (whereby said enzymatic reaction is enhanced).

The activator may be a substrate for an enzyme or it may not be a substrate for an enzyme in which case it will usually be an effector or other substance which can cause the enzyme to operate.

The facilitator may be produced as a direct product of the enzymatic reaction or may be produced as a product of a further reaction initiated by the direct product of the enzymatic reaction.

The facilitator produced by the enzymatic reaction is desirably the same as the activator so that a simple system results.

In one favoured form the activator and facilitator are both ATP

An alternative favoured system employs an activator which is a derivative of the facilitator. In such systems the activator is envisaged as a ligand or receptor labelled with the facilitator. In this aspect the skilled worker will appreciate that this aspect of the invention also provides a method of detecting said ligand or receptor labelled with    facilitator. Preferably the ligand or receptor labelled in this way is an antigen or antibody (when used herein the term "labelled" means chemically or physically associated in a manner which will not prevent the activation of the enzyme by the thus formed activator).

In yet another system the activator is different from the facilitator and is not a ligand or receptor labelled with a facilitator.

Favoured facilitators for use in this invention include pyridine nucleotides and ligands or receptors labelled with a pyridine nucleotide, preferred facilitator pyridine nucleotides include ATP and NAD. Preferred ligands and receptors labelled with a pyridine nucleotide include antigens and antibodies labelled with ATP or NAD. British Patents Nos.1548741 and 1552607 disclose ligands or receptors labelled with pyridine nucleotides which may be employed as activators in this invention (however said patents did not disclose the use of a system in which amplification was achieved by use of a facilitator which fed back into the assay)..

A favoured example of a method of detecting an activator according to this invention is the detection of NAD or a ligand or receptor labelled with NAD which comprises contacting said activator with NAD-depleted diaphorase, NADH and other required substrate (such as oxidized lipoamide or oxidized lipoic acid) characterized in that said reaction causes a detectable change (the removal of NADH) and the production of a facilitator (NAD).

A favoured example of a method of detecting an activator according to this invention is the detection of ATP or a ligand or receptor labelled with ATP which comprises contacting said activator with PFK, F6P, PK(I), ADP, PEP whereby F6P is converted by PFK to FDP which causes PK(I) to convert ADP to ATP and PEP to pyruvate which ATP is the facilitator and which conversion of PEP to pyruvate is the detectable change.

(The conversion of PEP to pyruvate can be detected by a method as hereinbefore described, for example by reaction with a hydrazine or by a further enzymatic change).

The direct product of the preceeding activated enzyme is FDP. The thus produced FDP then triggers the PK(I) to produce a detectable change.

A particularly simple way of carrying out the method of this invention is to have the above reagents in an appropriate cuvette of a spetrophoto-meter and to add the solution possibly containing the material to be detected. If the material to be detected is not present no change occurs but if it is present the change may be observed.

-14-

If desired the use of a spectrophotometer can be obviated in systems producing pyruvate (such as the kinase systems hereinbefore described) by detecting the formation of the reaction product between pyruvate and a hydrazine such as 2,4-dinitrophenylhydrazine which gives rise to a visually (or machine) detectable coloured product when the pH is subsequently rendered alkaline (for example with sodium hydroxide solution).

As previously explained, the method of this invention is usefully employed in investigating enzymes in biological samples. Unfortunately biological fluids, such as blood, serum and urine can contain a number of reactive species. Clearly it would be desirable to adapt the method of this invention to work with small samples of fluid possibly containing only small amounts of enzymes. Although this will employ a purification step, the increased sensitivity of this method is such as to render losses during purification acceptable.

Accordingly in a further aspect this invention provides a method of detecting an enzyme in a liquid which method comprises (i) contacting the liquid with a support to which is attached a substance to which the enzyme will bind specifically without eliminating its enzymatic properties, so that said enzyme becomes associated with said support , (ii) separating the support with its associated enzyme from the liquid and (iii) using the support with its associated enzyme in a detection method of this invention as hereinbefore described.

This aspect of the invention is most suitably adapted to the detection of an enzyme in blood, serum, urine, tissue extract or microbiological extract. Since enzymes are often used as markers in the diagnosis of human pathological conditions it follows that the method of this invention is preferably adapted to the detection of an enzyme in human blood, serum or urine. Of course use may be made of this invention in the vetinary field in analogous manner.

The support used in this invention may be in the form of a discrete surface such as that of a stick or

0049606

-16-

dish or filter system or in the form of small parti-
cules such as a powder or in the form of gel materials.
Aptly the support may be polystyrene, nylon, cross
linked dextrans (such as sephadex. and sepharose)
agarose , polyacrylamide, bromacetylcellulose or the
like. Such materials are presently known for the immobi-
lization of non-enzymatic biological materials such
as proteins, for example antigens, nucleic acids and
the like. The skilled worker will appreciate that such
supports can bind a wide range of materials so that,
in conventional manner, in order to prevent uptake of
unwanted enzymes or the like from the liquid the support
will be neutralised to prevent this, for example by
treatment with innocuous protein after the substance
to which the enzyme to be detected is bound to the support.

The substance bound to the support will normally
and preferably be an antibody to the enzyme with the
proviso that the antibody employed will not be one that
eliminates the enzymatic properties of the enzyme to
be detected. Very desirably the antibody will be a
monoclonal antibody.

The binding of the antibody (or other substance)
to the support may be physical or chemical binding.
Physical binding includes absorption onto the surface of the support and chemical binding may be ionic or covalent. Sandwich systems are also envisaged. The antibody may be bound in any manner as long as it is still capable of specifically binding the enzyme in a manner which will not eliminate the enzyme activity.

Alternatively the substance to be bound to the support may be some other substance with specific affinity for the enzyme in question, such as a substrate, substrate-analogue or inhibitor substance for that enzyme. Such approaches are possible with enzymes having more than one active enzymic site per enzyme molecule for - if only one site is taken up in binding to the support through the substrate, substrate analogue, inhibitor - the other site may still be available for catalytic activity.

Generally sufficient binding of the enzyme to the support with its bound substance will take place within 10 seconds to 30 minutes depending on the reaction parameters, concentrations employed and degree of accuracy required. More usually the binding will take place within 30 seconds to 10 minutes and preferably from 1 to 5 minutes. Mild agitation may be employed to facilitate the binding.

At the end of the binding stage the liquid may be separated and the support with the enzyme bound thereto washed. Generally it is preferred that this washing is thorough in order to remove unwanted unbound material. Washing may be carried out with water, saline, buffer or the like of which phosphate buffered saline is favoured. Suitably the washing liquid may include a mild surfactant such as a Tween.

At this point the support with the enzyme bound thereto may be promptly used in an assay for the enzyme.

In another and important aspect this invention provides a diagnostic test which comprises carrying out a method of this invention as hereinbefore described on a biological fluid or on material obtained from a biological fluid as hereinbefore described.

Desirably this aspect of the invention comprises a diagnostic test for breast cancer employing creatine kinase as hereinbefore described.

Aptly this aspect of the invention comprises a diagnostic test for muscle damage and also for liver disease employing pyruvate kinase as hereinbefore described.

In this specification the following symbols and abbreviations have the following meanings:

indicates activation of an enzyme.

indicates a feed-back of facilitator into the reaction sequence.

| | |
|---|---|
| AMP | adenosine 5'-monophosphate |
| ADP | adenosine 5'-diphosphate |
| ATP | adenosine 5'-triphosphate |
| GTP | guanosine 5'-triphosphate |
| CTP | cytidine 5'-triphosphate |
| UTP | uridine 5'-triphosphate |
| F6P | fructose-6-phosphate |
| FDP | fructose-1,6-diphosphate |
| PEP | phospho(enol)pyruvate |

| Pyr | pyruvate |
| --- | --- |
| CP | creatine phosphate |
| C | creatine |
| NAD | nicotinamide adenine dinucleotide |
| NADH | reduced nicotinamide adenine dinucleotide |
| NAC | N-acetyl cysteine |
| LDH | lactic dehydrogenase |
| PFK | phosphofructokinase |
| CK | creatine kinase |
| PK | pyruvate kinase |
| PK(I) | pyruvate kinase activatable by FDP[§] |
| PK(II) | pyruvate kinase not activatable by FDP[§] |
| EDTA | ethylenediaminetetraacetic acid |
| DMG | dimethyl-glutamate |

[§] as described by M. Malcovati, G. Valentini, & H.L. Kornberg in Acta vitamin. enzymol. (Milano) 1973, 27, 96. or enzymes of equivalent properties.

The following examples illustrate the invention. The following description illustrates the preparation of useful reagents.

Demonstration

Preparation of Pyruvate Kinase (I) for the examples


Cultures of E.coli strain K1-1 were grown on a synthetic medium containing essential nutrients and glycerol as carbon source, until well into their logarithmic phase of growth. The cells were harvested, washed by centrifugation and resuspended in a sonication buffer of 5mM phosphate, 1mM EDTA, 2mM mercaptoethanol pH 7.5 in an amount of approximately 20mg dry weight per ml. They were then disrupted with an MSE ultrasonic disintegrator for 4 minutes (in 30 second bursts while cooled). The resulting crude extract was separated from cell debris by centrifugation. As well as containing pyruvate kinase (I) this extract also contained an interfering isoenzyme with different properties, and also a number of other interfering enzyme activities. It was found, however, that it was possible to remove these contaminants by heating the extract to 55°C for 20-60 for example 45 minutes. This is the source of pyruvate kinase (I) referred to in the following examples.

Example 1

Detection of Pyruvate kinase (II)

In order to demonstrate the enhancement in sensitivity brought about by the method of this invention an assay for pyruvate kinase (II) was carried out with and without a secondary system to produce amplification. The two assays may be represented as follows:

Assay without Amplification

$$
\begin{array}{l}
\text{PEP} \searrow \quad \nearrow \text{ADP} \\
\qquad \text{PK(II)} \\
\text{Pyr} \nearrow \quad \searrow \text{ATP} \\
\\
\text{NADH} \searrow \\
\qquad \text{LDH} \\
\text{NAD} \nearrow \quad \text{lactate}
\end{array}
$$

Assay with amplification

$$
\begin{array}{l}
\text{PEP} \searrow \quad \nearrow \text{ADP} \\
\qquad \text{PK(II)} \\
\text{Pyr} \nearrow \quad \text{ATP} \searrow \quad \nearrow \text{F6P} \\
\qquad\qquad\qquad \text{PKF} \\
\qquad\qquad \text{ADP} \nearrow \quad \searrow \text{FDP} \\
\\
\text{ADP} \searrow \quad \nearrow \text{PEP} \\
\qquad \text{PK(I)} \\
\text{ATP} \nearrow \quad \text{Pyr} \searrow \quad \nearrow \text{NADH} \\
\qquad\qquad\qquad \text{LDH} \\
\qquad\qquad \text{lactate} \nearrow \quad \searrow \text{NAD}
\end{array}
$$

As PK(I) can in the absence of FDP be activated by a high enough concentration of PEP, it is necessary to limit the PEP concentrations used to those which will not cause spontaneous activation, while maintaining a sufficient substrate concentration for the enzyme. A concentration of 1mM has been found to satisfy these requirements under the conditions employed so far.

In the assay system without amplification PK(II) converts PEP and ADP into pyruvate and NADH to NAD and lactate. The change in concentration of NADH can be monitored by absorbence at 340nm.

In the assay system with amplification ( that is the assay incorporating the secondary system) the preceeding sequence takes place but in addition the secondary system generates ATP. In this amplified system, in addition to the PEP, ADP, NADH, PK(II) and LDH present in the non-amplified system, the following are also present: F6P, PFK and PK(I). In the presence of ATP (generated by catalyst PK II) the PFK converts the F6P into FDP. The FDP activates the PK(I ) which then is available to convert the PEP and ADP to ATP and pyruvate. The ATP thus produced feeds back within the secondary system and the pyruvate is converted into lactate by LDH in the presence of NADH. This end reaction is monitored at 340nm as in the unamplified assay.

To indicate the usefulness of this amplification method the PK(II) was initially assayed by the direct reaction as follows: The assay mix was as shown in Table 6 (left column) with a buffer consisting of 10nM dimethyl-glutamate (DMG) pH6.8 5mM $MgCl_2$. All assays were carried out at $30^{\circ}C$. The PK(II) was obtained from Calbiochem and, as a result of trials, a standard amount chosen which gave the activity shown in the Table. This amount was used for all following experiments. PK(I) was as in previous experimen

The assay system including the positive feed-back amplifier was composed as shown on the right hand column of Table 6. The activity attribuatable to the addition of the same amount of PK(II) as before and after allowing the feedback system to get underway for seven minutes, is shown. The activity attribuable to PK(II) is raised eleven times over the direct method.

### Table 6

| | Direct assay | Feed-back assay |
|---|---|---|
| ADP | 0.4mM | 0.4mM |
| PEP | 1.0mM | 1.0mM |
| NADH | 0.1mM | 0.1mM |
| LDH | 1.1 U | 1.1 U |
| PK(II) | standard | standard |
| F6P | | 2.0mM |
| PFK | | 1.0 U |
| PK(I) | | 10 µl |
| Buffer | To 1.0ml | To 1.0ml |
| Rate of Change Absorbence (OD Units at 340nm per 10 minutes) | 0.005 | 0.055 |

Using the direct method, the standard amount of PK(II) could just about be detected whereas it was quite readily detectable using the feed-back amplifier.

_____

### Example 2

Detection of ATP

In this example the same basic system described in Example 1 was again used, but instead of the system being used to detect an enzyme capable of giving rise to ATP, the pre-existing ATP in a solution was assayed, using a PK(I) extract which had been prepared by heating at $55^{o}C$ for 60 minutes.

As in the previous example, the assays were carried out in quartz spectrophotometer cuvettes for use with 1.0ml assay mixtures. They were made to contain:

| | |
|---|---|
| ADP | 0.4mM |
| PEP | 1.0mM |
| NADH | 0.1mM |
| LDH | 1.1U |
| F6P | 2.0mM |
| PK(I) | 10ul |
| PFK | 1.0U |
| material to be assayed | |
| buffer | to 1ml |

All dilutions and solutions were made in this same buffer.

The buffer was 10mM dimethyl-glutarate pH 6.8 and containing 5mM $MgCl_2$.

The test solutions were made to give a final ATP concentration in the cuvette of: 0.4mM; 0.05mM; 0.01mM; 0.001mM; 0.0001mM; control - with no ATP. On addition of a sample to the cuvette containing the above components the contents were mixed and the oxidation of NADH followed at 340nm and $30^oC$ with a spectrophotometer. The higher concentrations of ATP gave rise to an earlier initiation of observable NADH oxidation and subsequently increased in their rate faster than the lower concentrations. The method therefore has utility both in showing the presence of very low concentrations of ATP and also in demonstrating concentration.

Example 3

Detection of Creatine Kinase

The basis for this example is the same as that for with pyruvate kinase described in Example 1. Using the same system creatine kinase can be detected in the presence of its substrate creatine phosphate as then the creatine kinase gives rise to ATP from the ADP present and this activates the system as described in the previous example. The assay conditions themselves are in part based upon those described in Methods in Enzymatic analysis pages 789-793, edited by Hans Ulrich Bergmeyer and published by Verlag Chemie Gmbh, Weinheim, 1974.

For determination of creatine kinase a quartz cuvette for use with 1.0ml assay mixtures is made to contain a solution of the following composition:

| ADP | 0.4mM |
|------|-------|
| PEP | 1.0mM |
| NADH | 0.1mM |
| LDH | 1.1 U |
| F6P | 2.0mM |
| PFK | 1.0 U |

PK(I)      10ul

MgCl$_2$    10mM


CP         5mM

CK         material assayed


The mixture is combined in DMG buffer to a final volume of 1.0ml, pH 6.8 and a DMG concentration of 25mM. On addition of the material to be assayed the contents of the cuvette are mixed and the oxidation of NADH followed spectrophotometrically at 340nm and 30°C as before. Ten-fold dilutions of creatine kinases (creatine phosphokinases) obtained from the Sigma London Chemical Company (Type I from rabbit muscle, C3755, Type III from bovine heart, C7886 and Type IV from rabbit brain, C6638) are made to provide final concentration of from 1U to 0.001U in the assay mixture when added. Creatine kinase specific activation of the system leads to NADH oxidation, in an analogous manner to the previous example and is dependent on CK concentration.

Example 4

Detection of Creatine Kinase:

Example 3 may be repeated including 10mM of NAC. (This acts as an thiolic activating agent for the enzyme and aids in increasing the sensitivity of the system.)

Example 5

Detection of Creatine Kinase in suspected presence of myokinase:

Myokinase is sometimes a minor contaminent of enzymes such as PK(I). In order to allow the assay of examples 3 or 4 to take place in the presence of trace amounts of myokinase a sufficiency of AMP (for example 10mM) may be employed in the assay. (Inclusion of AMP in assays involving biological fluids is similarly desirable for this reason).

Example 6

Five hundred units of creatine phosphokinase from bovine heart and obtained from the Sigma (London) Chemical Company Ltd (Type III catalogue number C7886) was dissolved in 1ml of the standard buffer used i the assay - namely: 10mM dimethyl-glutarate buffer at pH 6.8 and containing 5mM MgCl$_2$ and 0.1% in bovine serum albumin (Sigma London catalogue number A6003). Three ten-fold serial dilutions of this enzyme solution were th made using the same buffer. Ten micro-litre aliquots of each of the dilutions were then tested in standard mixtures of the following composition for their ability to form ATP and thus activate the system.

| | |
|---|---|
| ADP | 0.4mM |
| PEP | 1.0mM |
| NADH | 0.1mM |
| F6P | 2.0mM |
| CP | 4.0mM |
| LDH | 1 enzyme unit |
| PFK | 1 enzyme unit |
| PK(I) | 10ul standard preparation |

The LDH was obtained from The Boehringer Corporation (Lo Ltd - Cat. No. 127884 (from rabbit muscle). All other components except the standard PK(I),which was prepared previously described,were obtained from the Sigma (Londo Chemical Company and were as follows: ADP Grade 1 Cat. N

A 0127; PEP monopotassium salt Cat. No. P7127; NADH
dipotassium salt Cat. No. N 4505; FGP disodium salt
Grade 1 Cat. No. F 3627; CP disodium salt hydrate Cat.
No. P 6502; PFK Type III from rabbit muscle Cat. No.
F 6877.

 

The complete assay mixture for each test was
made to 1ml with the standard buffer in a suitable quartz
spectrophotometer cuvette at $30^{\circ}C$. On addition of the final
component (the creatine phosphokinase to be assayed) the
contents of the cuvette were well mixed and the oxidation
of NADH followed by continuous monitoring at 340nm. The
rate of change in optical density at two minutes after
initiation was taken for each test and used to compare
the activities as follows:

| Units of CK in final assay mixture | O.D. Units (340nm) change/minute at 2 minutes |
|---|---|
| 0.05 | 0.0216 |
| 0.005 | 0.0068 |
| nil | 0.0017 |

Enzyme Units where quoted in the text are those
units used by the particular manufacturers for the enzymes
they have supplied.

Example 7

This example uses the same reagents and conditions as Example 6, however, instead of showing the dependence of the system on the enzyme creatine kinase in the presence of its substrate creatine phosphate it shows that the system can be made dependent on the presence of the substrate creatine phosphate if creatine kinase is present as a reagent.

Mixtures were made up in 10$\underline{m}$M dimethyl-glutamate buffer at pH 6.8 and containing 5$\underline{m}$M MgCl$_2$ and 0.1% in bovine serum albumin as follows:

| | | | |
|------|--------|--------|--------|
| ADP | 0.4$\underline{m}$M | 0.4$\underline{m}$M | 0.4$\underline{m}$M |
| PEP | 1.0$\underline{m}$M | 1.0$\underline{m}$M | 1.0$\underline{m}$M |
| NADH | 0.1$\underline{m}$M | 0.1$\underline{m}$M | 0.1$\underline{m}$M |
| F6P | 2.0$\underline{m}$M | 2.0$\underline{m}$M | 2.0$\underline{m}$M |
| LDH | 1 U | 1 U | 1 U |
| PFK | 1 U | 1 U | 1 U |
| PK(I) | 10ul | 10ul | 10ul |
| CK | 0.05 U | 0.05 U | 0.05 U |
| CP | 4.0$\underline{m}$M | 0.4$\underline{m}$M | nil |

In each case the components were combined from stock solutions in a quartz cuvette of 1ml capacity, mixed and immediately monitored for NADH oxidation by following their optical density at 340nm and 30$^o$C. The rate of change of absorbance at 1.5 minutes after mixing was

0049606

found to be as follows:

| CP concentration | O.D. Units (340nm) change/minute at 1.5 minutes |
|---|---|
| 4.0mM | 0.0118 |
| 0.4mM | 0.0070 |
| nil | 0.0011 |

The system was seen to be dependent on the presence of creatine phosphate and the concentration thereof.

CLAIMS

1.      A method of detecting a catalyst or its substrate
in a catalysable reaction which comprises carrying out
an assay for the catalyst or its substrate in a manner
which produces a product characterised in that said
product is an activator for an enzymatic reaction which
is activated by the production thereof so as to effect a
detectable change and to produce a facilitator for the
enzymatic reaction; whereby said enzymatic reaction is
enhanced.

2.      A method as claimed in claim 1 wherein the
facilitator produced by the enzymatic reaction is the
same as the activator produced by the assay for the
catalyst or its substrate.

3.      A method as claimed in claim 2 adapted to the
detection of an enzyme.

4.      A method as claimed in claim 3 wherein the
enzyme to be detected is a kinase.

5.      A method as claimed in claim 4 wherein the kinase
is creatin kinase.

6.      A method as claimed in claim 4 wherein the kinase is pyruvate kinase.

7.      A method as claimed in any of claims 1 to 6 wherein the activator is ATP.

8.      A method as claimed in any of claims 4 to 6 wherein the kinase to be detected is being used as a diagnostic aid or to monitor the progress of a pathological state.

9.      A method of detecting an activator which comprises contacting said activator with an enzyme to be activated and all other components needed for the enzymatic reaction to proceed characterised in that said enzymatic reaction causes detectable change and the production of a facilitator; whereby said enzymatic reaction is enhanced.

10      A method as claimed in claim 9 wherein the activator is the same as the facilitator.

11.     A method as claimed in claim 10 wherein the activator and facilitator are both ATP.

12.    A method as claimed in claim 9 wherein the activator is a derivative of the facilitator.

13.    A method as claimed in any of claims 1 to 12 wherein the change to be detected is detection of a coloured material by visual means.

14.    A method of detecting an enzyme in a liquid which method comprises (i) contacting the liquid with a support to which is attached a substance to which the enzyme will bind specifically without eliminating its enzymatic properties, so that said enzyme becomes associated with said support, (ii) separating the support with its associated enzyme from the liquid and (iii) using the support with its associated enzyme in a method as claimed in any of claims 1 to 6, 8 and 13.

European Patent
Office

**EUROPEAN SEARCH REPORT**

Application number

EP 81 30 4542

| | DOCUMENTS CONSIDERED TO BE RELEVANT | | CLASSIFICATION OF THE APPLICATION (Int. Cl. 3) |
|---|---|---|---|
| Category | Citation of document with indication, where appropriate, of relevant passages | Relevant to claim | |
| XE | GB - A - 2 059 421 (COLIN HENRY SELF) <br><br> * Abstract; page 2, lines 36-63; page 3, lines 58-65; page 4, lines 1-15; page 5, lines 1-26; page 6, lines 1-23; page 7, lines 7-30; page 8, lines 3-24; page 9, lines 1-14; examples 1-2; claims 11, 15-16, 19,23-24 * <br> -- | 1-14 | C 12 Q 1/00 <br> 1/48// <br> G 01 N 33/54 |
| E | WO - A - 81/00725 (THE PRINCE CHARLES HOSPITAL DEBELOPMENT CENTRE TRUST) <br><br> * Abstract; page 3, lines 1-28; page 4, lines 3-31; page 5, lines 1-29; page 6, lines 19-35; page 7, lines 7-27; claims 1,7-9 * <br> -- | 1-13 | **TECHNICAL FIELDS SEARCHED (Int.Cl.3)** <br><br> C 12 Q 1/00 <br> 1/32 <br> 1/48 <br> 1/50 <br> G 01 N 33/54 |
| P | GB - A - 2 055 200 (SEPPO KOHLE-MAINEN) <br><br> * Abstract; Figures 1-6; page 2, lines 7-60; page 3, lines 1-21; claims 1-11 * <br> -- | 1-13 | |
| D | GB - A - 1 552 607 (MILES LABORATORIES) <br><br> * Page 3, lines 10-46; page 10, lines 15-29; page 11, tables B,C; page 12, Table D; page 13, table D; page 14, lines 1-12, 26-42; claims 1,11 * <br> -- | 1-13 | **CATEGORY OF CITED DOCUMENTS** <br><br> X: particularly relevant if taken alone <br> Y: particularly relevant if combined with another document of the same category <br> A: technological background <br> O: non-written disclosure <br> P: intermediate document <br> T: theory or principle underlying the invention <br> E: earlier patent document, but published on, or after the filing date <br> D: document cited in the application <br> L: document cited for other reasons |
| D | GB - A - 1 548 741 (MILES LABO-RATORIES) <br><br> ./. | 1-13 | |

&: member of the same patent family, corresponding document

| The present search report has been drawn up for all claims | | |
|---|---|---|
| Place of search <br> The Hague | Date of completion of the search <br> 07-01-1982 | Examiner <br> DE LUCA |

EPO Form 1503.1 08.78

European Patent
Office

**EUROPEAN SEARCH REPORT**

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | * Page 3, lines 1-31; page 5, lines 15-37; page 8, lines 5-19; page 9, tables C.D; page 10, table D; page 11, table D; page 12, lines 12-39; claims 1,8-9 * | | |
| | -- | | |
| DA | CHEMICAL ABSTRACTS, vol. 81, no. 17, October 28, 1974 page 185, ref. 101156h Columbus, Ohio, US MALCOVATI, M. et al. "Two forms of pyruvate kinase in Escherichia coli. Their properties and regulation" & ACTA VITAMINOL. ENZYMOL. 1973, 27 (1-4), 96-111 * Whole abstract * | 6 | TECHNICAL FIELDS SEARCHED (Int. Cl.³) |
| | ---- | | |

EPO Form 1503.2  06.78